# FASCICULE DE BREVET EUROPEEN

(11) **EP 4 483 931 B1**
(45) Date de publication et mention de la délivrance du brevet: **17.12.2025**
(21) Numéro de dépôt: 24179098.9
(22) Date de dépôt: 30.05.2024
(51) Int. Cl.: A61M 16/00, A61M 16/08, A61M 16/12, A61M 16/20, G01N 33/00

(54) **AFFICHAGE D'UN RAPPEL DE MAINTENANCE SUR UN APPAREIL DE FOURNITURE DE MONOXYDE D'AZOTE MÉDICAL**
ANZEIGE EINES WARTUNGSERINNERUNGSRÜCKRUFS AUF EINEM MEDIZINISCHEN STICKOXIDBEREITSTELLUNGSGERÄT
DISPLAY OF MAINTENANCE REMINDER ON MEDICAL NITRIC OXIDE DELIVERY APPARATUS

(30) Priorité: 29.06.2023 FR 2306904
(43) Date de publication de la demande: 01.01.2025
(73) Titulaire: INOSYSTEMS, 92160 Antony (FR)
(72) Inventeur: BLANDIN, Yann, 92160 Antony (FR); SCHMITT, Mary, 92160 Antony (FR); MARCHAL, Frédéric, 92160 Antony (FR); PROUVEZ, Nathan, 92160 Antony (FR)
(74) Mandataire: Air Liquide

(56) Documents cités:
- EP-A1- 2 581 103
- EP-A2- 0 839 546
- US-A1- 2022 106 189

## Description

L'invention concerne un appareil ou dispositif de fourniture et monitorage d'un gaz contenant du monoxyde d'azote (NO), en particulier d'un mélange gazeux NO/N₂, couramment appelé appareil de fourniture de NO, utilisé pour traiter une ou des personnes souffrant d'hypertension artérielle pulmonaire aiguë, configuré pour afficher un rappel de maintenance préférentiellement journalier

Le monoxyde d'azote inhalé (NOi) est un traitement de référence pour traiter les personnes, i.e. les patients, souffrant d'hypertension artérielle pulmonaire aiguë. En effet, lorsqu'il est inhalé, le NO dilate les vaisseaux pulmonaires et augmente l'oxygénation en améliorant les échanges gazeux. Ces propriétés sont utilisées pour traiter différentes conditions médicales, comme l'Hypertension Artérielle Pulmonaire du Nouveau-né ou PPHN (pour *Persistent Pulmonary Hypertension of the Newborn),* le Syndrome de Détresse Respiratoire Aigüe ou SDRA observé principalement chez l'adulte ou les hypertensions pulmonaires (HP) en chirurgie cardiaque chez l'adulte ou l'enfant, comme décrit notamment par EP-A-560928, EP-A-1516639 et US-A-10,201,564.

Usuellement, une faible quantité de NO gazeux (i.e. quelques ppm vol.), dilué dans de l'azote (N₂) est injectée et diluée dans un flux gazeux contenant de l'oxygène, typiquement au moins 20 à 21%vol. environ d'oxygène (O₂), tel un mélange N₂/O₂ ou de l'air, voire de l'oxygène pur, qui est véhiculé par le circuit patient d'une installation de fourniture de gaz, et le mélange gazeux final obtenu contenant du NO et de l'oxygène est ensuite inhalé par le patient. La concentration de NO finale, qui correspond à une posologie, est déterminée par le médecin ou analogue. En général, elle est comprise entre 1 et 80 ppm en volume (ppmv), typiquement de l'ordre de 10 à 20 ppmv, en fonction de la population traitée, i.e. nouveau-nés, enfants, adolescents ou adultes, et de la maladie à traiter, dans le gaz final NO/N₂/O₂ administré par inhalation au patient considéré, c'est-à-dire après injection du mélange NO/N₂ dans le flux de gaz contenant de l'oxygène (i.e. environ >21 %vol.) véhiculé par le circuit patient.

La mise en œuvre d'un traitement par NOi comprend habituellement une ou des bouteilles de mélange NO/N₂ (ou un générateur de NO), un appareil de fourniture et monitorage de NO, un ventilateur médical et un kit patient comprenant un circuit patient et une interface respiratoire, telle une sonde d'intubation trachéale..., voire d'autres éléments, tel un humidificateur de gaz ou autre. De telles installations sont notamment décrites par US2022/106189, EP2581103 et EP0839546.

Tous ces matériels doivent être prêts pour un démarrage urgent du traitement par NOi, dès la prescription du médecin. Sachant que le temps de traitement d'un patient peut être de quelques heures à plusieurs jours, il est primordial de contrôler très régulièrement leur bon fonctionnement afin de garantir le bon déroulement du traitement et ce, dans des conditions de sécurité optimale pour le patient. En effet, toute interruption inattendue de traitement expose le patient à des risques graves pouvant mettre en jeu sa survie.

Ainsi, vérifier le bon fonctionnement de l'appareil de fourniture de NO est particulièrement important car il permet de monitorer et de contrôler/ajuster la quantité de NO fournie au patient pendant les soins par NOi. En particulier, il est primordial de calibrer régulièrement les cellules de monitorage ou d'analyse, c'est-à-dire les capteurs de NO, NO₂ et O₂ servant à opérer les mesures de teneur en NO, NO₂ et la fraction inspirée en oxygène FiO₂ dans le gaz administré au patient afin de s'assurer une précision des mesures opérées par ces cellules, typiquement de la dose de NO administrée au patient et l'absence (ou quasi-absence) de NO₂ toxique et la proportion en oxygène. Le maintien de la précision de ces mesures est un point majeur de sécurité.

Or, les équipes de soin (i.e. médecins, infirmières ou autres personnels soignants) travaillant dans les services de réanimation et de chirurgie des établissements hospitaliers, où est le NOi est administré, enchaînent quotidiennement les soins et les procédures médicales, ce qui peut conduire à un oubli de certaines tâches de contrôle.

Un problème est dès lors de pouvoir minimiser le risque que les équipes de soin utilisant du NOi pour traiter les patients en ayant besoin, omettent d'opérer tout ou partie des vérifications indispensables du bon fonctionnement de l'appareil de fourniture de NO, c'est-à-dire de l'appareil délivrant le flux gazeux contenant du NO, en particulier une calibration régulière des cellules de monitorage, c'est-à-dire des capteurs de NO, NO₂ et O₂

Une solution de l'invention concerne alors un appareil de fourniture (i.e. de délivrance) d'un gaz contenant du NO, aussi appelé appareil de fourniture de NO ou appareil de délivrance de NO, comprenant :
- un circuit de gaz interne, e.g. un circuit de gaz interne, pour acheminer un flux de gaz contenant du NO, comprenant des moyens à valve,
- des moyens de pilotage à microprocesseur, i.e. un contrôleur ou analogue,
- un afficheur graphique tactile commandé par les moyens de pilotage, et
- une ligne d'analyse de gaz et des cellules de mesure comprenant au moins un capteur relié électriquement aux moyens de pilotage.

De plus, les moyens de pilotage sont configurés pour commander, à une fréquence temporelle donnée, un affichage temporaire sur l'afficheur graphique d'au moins une première touche de sélection virtuelle dont l'actionnement par un utilisateur engendre un lancement par les moyens de pilotage, d'une procédure de calibration du ou des capteurs de la ligne d'analyse de gaz.

Selon le mode de réalisation considéré, l'appareil de l'invention peut comprendre l'une ou plusieurs des caractéristiques suivantes :
- le circuit de gaz interne comprend un ou plusieurs passages de gaz.
- la ligne d'analyse de gaz est aussi appelée « ligne de calibration », « ligne de monitorage » ou « ligne d'échantillonnage ».
- l'appareil de l'invention est configuré pour opérer un monitorage et une fourniture de NO gazeux, i.e. de gaz contenant du NO, tel un mélange NO/N₂,
- la procédure de calibration des capteurs débute après actionnement par l'utilisateur de la première touche de sélection virtuelle.
- la première touche de sélection virtuelle coopère avec les moyens de pilotage, pour leur fournir au moins un signal de commande correspondant à un choix opéré par l'utilisateur correspondant au lancement de la procédure de calibration des capteurs.
- les moyens de pilotage sont en outre configurés pour commander un affichage sur l'afficheur graphique d'au moins une information relative à un ou plusieurs contrôles de sécurité à réaliser, notamment la procédure de calibration des capteurs.
- il comprend des moyens de mémorisation, telle une mémoire informatique, notamment une mémoire flash ou autre.
- la ou les procédures de contrôle de sécurité sont mémorisées au sein des moyens de mémorisation, en particulier la procédure de calibration des capteurs.
- les moyens de mémorisation sont configurés pour mémoriser la ou les procédures de contrôle de sécurité, en particulier la procédure de calibration des capteurs
- les procédures de contrôle de sécurité comprennent la procédure de calibration du ou des capteurs de la ligne d'analyse de gaz.
- les moyens de pilotage sont configurés pour commander l'affichage de ladite au moins la première touche de sélection virtuelle à une fréquence temporelle de 1 à 4 fois par jour, de préférence encore 1 ou 2 fois par jour.
- les moyens de pilotage sont configurés pour commander l'affichage de ladite au moins la première touche de sélection virtuelle à une fréquence temporelle d'au moins 1 fois par jour :
   ∘ soit à heure fixe, par exemple tous les jours à 08:00 du matin,
   ∘ soit lors de la mise en route de l'appareil, c'est-à-dire au démarrage de l'appareil,
   ∘ soit après une durée prédéfinie après la démarrage de l'administration du dispositif, par exemple 24h après l'initiation du traitement.
   ∘ soit après une durée prédéfinie après la dernière calibration opérée, par exemple 24h après la dernière calibration ayant été réalisée.
- les moyens de pilotage sont configurés pour commander, en outre, un affichage d'une seconde touche de sélection virtuelle, i.e. une seconde touche tactile, simultanément ou successivement à ladite première touche de sélection virtuelle.
- ladite seconde touche de sélection virtuelle est configurée pour annuler ou refuser ladite procédure de calibration des capteurs, en réponse à un actionnement par l'utilisateur de la seconde touche de sélection virtuelle.
- la seconde touche de sélection virtuelle coopère avec les moyens de pilotage, pour leur fournir au moins un signal de commande correspondant à un choix opéré par l'utilisateur correspondant d'annuler ou de refuser ladite procédure de calibration, i.e. de la différer.
- les moyens de pilotage sont configurés pour commander un affichage simultané de ladite au moins une information et des première et éventuellement seconde touches de sélection virtuelles.
- les moyens de pilotage sont configurés pour commander un affichage au sein d'une fenêtre d'affichage éphémère, de ladite au moins une information et de la première touche de sélection virtuelle et éventuellement de la seconde touche de sélection virtuelle.
- les moyens de pilotage sont en outre configurés pour commander un affichage sur l'afficheur graphique d'une troisième touche de sélection virtuelle dont l'actionnement par l'utilisateur engendre un arrêt d'une procédure de calibration ayant débuté, c'est-à-dire ayant commencé après appui par l'utilisateur sur la première touche de sélection virtuelle.
- les moyens de pilotage sont en outre configurés pour commander un affichage sur l'afficheur graphique d'un barographe, d'un sablier ou d'un graphique temporel analogue représentant le temps s'étant écoulé depuis le début de la procédure de calibration et/ou le temps restant avant terminaison, i.e. fin, de la procédure de calibration.
- les moyens de pilotage sont en outre configurés pour commander un affichage sur l'afficheur graphique du graphique temporel représentant le temps s'étant écoulé depuis le début de la procédure de calibration et/ou le temps restant avant terminaison de la procédure de calibration, en particulier un graphique temporel représentant un barographe ou un sablier.
- les moyens de pilotage sont en outre configurés pour opérer un affichage simultané sur l'afficheur graphique, de la troisième touche de sélection virtuelle et du graphique temporel, c'est-à-dire que l'affichage de la troisième touche de sélection virtuelle et celui du graphique temporel, i.e. sablier, barographe ou analogue, sont opérés simultanément.
- il comprend un analyseur de gaz interne comprenant la ligne d'analyse de gaz et ledit au moins un capteur, i.e. un ou plusieurs capteurs, de préférence un capteur de NO et un capteur de NO₂, et préférentiellement un capteur d'O₂.
- le circuit interne permet d'acheminer le flux de gaz contenant du NO entre (au moins) une entrée de gaz et (au moins) une sortie de gaz fournissant le gaz contenant le NO, i.e. un mélange NO/N₂.
- le circuit interne fait partie d'un circuit de gaz agencé dans l'appareil de fourniture de NO.
- ledit au moins un capteur comprend une ou des cellules électrochimiques.
- il comprend un capteur de NO configuré pour mesurer une concentration (i.e. teneur ou dose) en NO.
- il comprend un capteur de NO₂ configuré pour mesurer une concentration (i.e. teneur ou dose) en NO₂.
- il comprend un capteur d'O₂ configuré pour mesurer une concentration (i.e. teneur ou dose) en O₂.
- le ou les capteurs sont agencés de manière à opérer des mesures de concentration au sein de la ligne d'analyse de gaz.
- ledit circuit interne comprend un ou des conduits, passages, tuyaux ou analogues.
- la ligne d'analyse de gaz comprend des conduits, passages, tuyaux ou analogues.
- les moyens de pilotage à microprocesseur comprennent (au moins) une carte électronique.
- les moyens de pilotage comprennent un (ou des) microprocesseur agencé(s) sur la carte électronique.
- l'afficheur graphique tactile fait partie d'une IGU ou interface graphique utilisateur.
- l'afficheur graphique est à affichage en couleurs.
- l'afficheur graphique est configuré pour détecter un contact d'un doigt d'un utilisateur appuyant sur la surface de l'afficheur graphique.
- l'afficheur graphique comprend une dalle tactile.
- la première et la seconde touches de sélection virtuelles sont des touches tactiles s'affichant sur l'afficheur graphique.
- la première et la seconde touches de sélection virtuelles coopèrent avec les moyens de pilotage.
- les moyens de pilotage agissent en réponse à tout appui digital d'un utilisateur sur l'une et/ou l'autre des première et la seconde touches de sélection.
- il comprend un compteur temporel.
- les moyens de pilotage intègrent ou coopèrent avec le compteur temporel.
- le compteur temporel est intégré au microprocesseur.
- l'affichage se fait automatiquement à la fréquence donnée, typiquement toutes les 24h, c'est-à-dire 1 fois par jour au minimum, en étant commandé par les moyens de pilotage qui coopèrent avec le compteur temporel.
- il comprend des moyens de sélection pour fixe, régler ou sélectionner une quantité de NO souhaitée, i.e. une teneur ou concentration en NO.
- les moyens de sélection comprennent au moins une touche ou un bouton, en particulier une touche virtuelle affichée sur l'afficheur graphique.
- les moyens à valve comprennent au moins une électrovanne, de préférence une ou des électrovannes proportionnelles.
- les moyens à valve, en particulier la ou les électrovannes, sont commandés par les moyens de pilotage pour contrôler le flux de gaz au sein dudit circuit de gaz interne.

L'invention porte aussi sur une installation d'administration de gaz à un patient, i.e. d'un gaz contenant du NO, comprenant :
- au moins une source de gaz contenant du NO gazeux, en particulier un mélange gazeux NO/N₂,
- un appareil de fourniture de gaz selon l'invention, alimenté en gaz contenant du NO par ladite au moins une source de gaz,
- un ventilateur médical pour fournir un gaz contenant de l'oxygène et
- une ligne d'alimentation alimentée en gaz par l'appareil de fourniture de gaz selon l'invention (i.e. en gaz contenant du NO, tel un mélange NO/N₂) et par le ventilateur médical (i.e. en gaz contenant de l'oxygène, e.g. de l'air ou un mélange O₂/N₂).

Selon le mode de réalisation considéré, l'installation d'administration de gaz de l'invention peut comprendre l'une ou plusieurs des caractéristiques suivantes :
- le ventilateur médical, c'est-à-dire un appareil d'assistance ventilatoire, est en communication fluidique avec la ligne d'alimentation pour alimenter ladite ligne d'alimentation avec un gaz respiratoire contenant au moins environ 21% vol. d'oxygène, en particulier de l'air ou un mélange N₂/O₂.
- le ventilateur médical est un appareil d'assistance respiratoire fournissant le gaz à pression constante ou, alternativement, le ventilateur médical est un ventilateur de type HFO (High Frequency Oscillations) délivrant le gaz par oscillations à haute fréquence.
- la ligne d'alimentation en gaz est alimentée en un gaz thérapeutique, de préférence un mélange NO/N₂, par l'appareil de fourniture de gaz et en un gaz respiratoire contenant au moins environ 21% vol. d'oxygène, de préférence de l'air ou un mélange N₂/O₂, par le ventilateur médical
- la source de gaz contient un mélange gazeux NO/N₂ contenant moins de 2000 ppm en volume de NO, le reste étant de l'azote, de préférence moins de 1000 ppm en volume de NO, le reste étant de l'azote, préférentiellement, la source de gaz thérapeutique contient un mélange NO/N₂ contenant de 250 à 900 ppm en volume de NO, le reste étant de l'azote, par exemple de l'ordre de 800 ppm en volume de NO, le reste étant de l'azote.
- elle comprend en outre un humidificateur de gaz agencé sur la ligne d'alimentation en gaz, de préférence en aval du site où le dispositif de fourniture de gaz thérapeutique est raccordé fluidiquement à ladite ligne d'alimentation en gaz de manière à l'alimenter en gaz thérapeutique.
- elle comprend en outre une ligne de récupération des gaz expirés par le patient.
- la ligne d'alimentation en gaz et la ligne de récupération des gaz expirés sont raccordées à une pièce de raccordement, de préférence une pièce en Y, et définissent ou forment tout ou partie d'un circuit patient.
- la ligne d'alimentation forme une branche inspiratoire du circuit patient.
- la ligne de récupération des gaz expirés forme une branche expiratoire du circuit patient.
- la ligne d'analyse de gaz de l'appareil de fourniture de NO est reliée fluidiquement à la ligne d'alimentation en gaz, i.e. la branche inspiratoire.
- la ligne d'alimentation en gaz, i.e. la branche inspiratoire, comprend un capteur de débit agencé entre le ventilateur et le site d'injection du gaz contenant le NO provenant de l'appareil de fourniture de NO.
- le capteur de débit est relié aux moyens de pilotage de l'appareil de fourniture de NO.
- le capteur de débit mesure le débit de gaz contenant de l'oxygène délivré par le ventilateur médical et circulant dans la ligne d'alimentation en gaz, i.e. la branche inspiratoire.
- la ligne d'alimentation en gaz, i.e. la branche inspiratoire, est raccordée fluidiquement à un port de sortie du ventilateur médical de manière à récupérer et acheminer le gaz délivré par le ventilateur médical.
- la ligne de récupération des gaz expirés, i.e. la branche expiratoire, est raccordée fluidiquement à un port d'entrée du ventilateur médical de manière à acheminer jusqu'au ventilateur médical tout ou partie des gaz expirés par le patient.
- au moins une source de gaz thérapeutique comprend un ou plusieurs récipients de gaz, en particulier une ou des bouteilles de gaz sous pression.
- le (ou les) récipient de gaz est équipé d'un robinet de distribution de gaz avec ou sans détenteur intégré (RDI).
- le robinet de distribution de gaz est en alliage de cuivre, tel du laiton, et/ou est équipé d'un capotage de protection agencé autour du robinet de distribution de gaz, par exemple en matériau polymère (i.e. plastique), en métal ou leurs combinaisons.
- le (ou les) récipient de fluide est (sont) une bouteille de gaz sous pression contenant, lorsqu'il est plein, un mélange gazeux, en particulier NO/N₂, à une pression d'au moins 150 à 200 bar abs, voire d'au moins 250 à 300 bar abs.
- le récipient de fluide a une forme générale cylindrique, en particulier d'ogive.

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- par « capteur», on entend un dispositif de mesure conçu pour et apte à et/ou configuré pour mesurer et fournir un ou des signaux ou mesures, par exemple un capteur de NO est un dispositif de mesure de concentration en NO permettant de mesurer et fournir un ou des signaux ou mesures de concentration en monoxyde d'azote.
- les termes « concentration », « dose » et « teneur » sont considérés comme équivalents.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à valves » peuvent être remplacés par « dispositif à valves », les « moyens de contrôle » peuvent être remplacés par « dispositif de contrôle »...

L'invention va maintenant être mieux comprise grâce à la description détaillée suivante, faite à titre illustratif mais non limitatif, en référence aux figures annexées parmi lesquelles :
Fig. 1 représente un mode de réalisation d'une installation d'administration de gaz, i.e. d'un gaz contenant du NO, comprenant un appareil de fourniture de NO selon l'invention.
Fig. 2 schématise le fonctionnement d'un appareil de fourniture de NO selon l'invention.
Fig. 3 schématise un exemple d'affichage d'une information relative à des contrôles de sécurité à réaliser et d'une première et d'une seconde touche de sélection virtuelle, sur l'afficheur graphique de l'appareil de fourniture de NO selon l'invention.
Fig. 4 schématise un exemple d'affichage d'une troisième touche de sélection virtuelle, sur l'afficheur graphique de l'appareil de fourniture de NO selon l'invention.

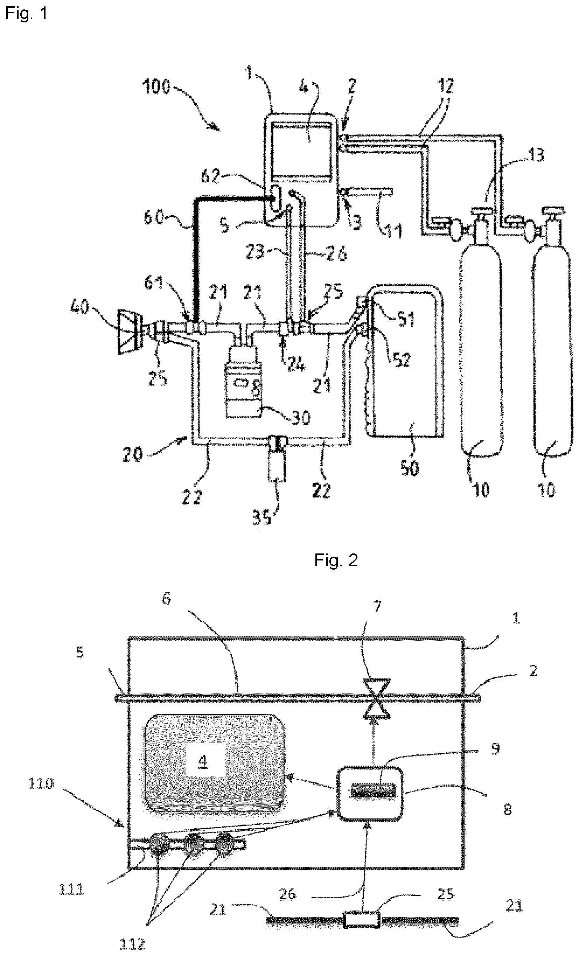

Un mode de réalisation d'une installation d'administration de gaz 100 est illustré sur Fig. 1. Elle comprend ici deux bouteilles de gaz sous pression ou sources de gaz 10 contenant chacune un mélange gazeux NO/N₂ contenant jusqu'à 1 % vol de NO, typiquement entre 100 et 1500 ppmv de NO (reste N₂), à savoir ici un mélange gazeux NO/N₂ contenant par exemple 450 ou 800 ppmv de NO (reste N₂), qui alimentent en mélange NO/N₂, un dispositif ou appareil de fourniture, i.e. de délivrance, de NO 1 permettant de monitorer/suivre et contrôler la fourniture du mélange gazeux NO/N₂.

Les bouteilles de gaz 10 sont reliées fluidiquement à l'appareil de fourniture de NO 1, via des lignes d'amenée de gaz 12, tel que des tuyaux ou conduits flexibles ou analogues, qui peuvent être équipées de dispositifs de régulation et/ou de suivi de la pression de gaz, tels que détendeur de gaz 13, manomètres... Les lignes d'amenée de gaz 12 sont reliées à une ou plusieurs entrées de gaz 2 du dispositif de délivrance de NO 1 qui alimentent un circuit de gaz interne, par exemple un ou plusieurs passages de gaz interne à l'appareil de délivrance de NO 1, servant à acheminer le gaz au sein du dispositif de délivrance de NO 1, c'est-à-dire dans le boitier ou la carcasse externe du dispositif de délivrance de NO 1.

Le dispositif de fourniture de NO 1 comprend aussi une entrée d'oxygène 3 reliée fluidiquement, via une ligne d'amenée d'oxygène 12, tel un tuyau flexible ou analogue, à une source d'oxygène par exemple une bouteille d'oxygène sous pression ou un réseau hospitalier, c'est-à-dire une canalisation d'alimentation en oxygène agencée dans un bâtiment hospitalier.

L'installation d'administration de gaz 100 comprend par ailleurs un ventilateur médical 50, c'est-à-dire un appareil d'assistance respiratoire, qui fournit un flux de gaz respiratoire contenant de l'oxygène, typiquement de l'ordre d'au moins environ 21% d'oxygène, tel de l'air ou un mélange oxygène/azote (N₂/O₂), voire de l'oxygène.

Le ventilateur médical 50 et l'appareil de fourniture de NO 1 de l'installation d'administration de gaz 100 sont en communication fluidique avec une ligne d'alimentation en gaz ou branche inspiratoire 21 d'un circuit patient 20. La ligne d'alimentation en gaz ou branche inspiratoire 21 sert à acheminer le flux gazeux vers le patient, lequel est formé par mélange du flux à base d'oxygène (i.e. air ou mélange NO/N₂) provenant du ventilateur médical 50 et du flux contenant le NO, i.e. le mélange gazeux NO/N₂, délivré par le dispositif de fourniture de NO 1.

Plus précisément, le dispositif de fourniture de NO 1 délivre ou injecte le mélange NO/N₂, par exemple à 450 ou 800 ppmv de NO, dans la ligne d'alimentation en gaz 21, via un conduit ou ligne d'injection 23 venant se raccorder à une sortie 5 de NO de l'appareil 1, de manière à mélanger (en 24.1) le flux de NO/N₂ au flux de gaz à base d'oxygène (avec au moins environ 21% d'O₂), e.g. de l'air ou un mélange oxygène/azote, délivré par le ventilateur médical 50 et véhiculé par la branche inspiratoire 21 du circuit patient 20 de sorte d'obtenir un mélange final contenant essentiellement du NO à la posologie désirée, de l'azote (N₂) et de l'oxygène (O₂), et éventuellement des impuretés inévitables (e.g. argon, CO₂, NO₂, ....), c'est-à-dire un mélange gazeux NO/N₂/O₂.

La branche inspiratoire 21 comprend en outre un humidificateur de gaz 30 agencé en aval du site 24 où se fait l'injection de NO dans la branche inspiratoire 21. Il permet d'humidifier le flux de gaz, e.g. le mélange gazeux NO/N₂/O₂, avant qu'il ne soit inhalé par le patient à traiter, au moyen d'une interface respiratoire 40, telle une sonde d'intubation trachéale, un masque respiratoire ou analogue.

Est prévue aussi une ligne de récupération des gaz expirés par le patient formant la branche expiratoire 22 du circuit patient 20. La ligne d'alimentation en gaz ou branche inspiratoire 21 et la ligne de récupération ou branche expiratoire 22 des gaz expirés sont raccordées à une pièce de raccordement 25, de préférence une pièce en Y.

La branche inspiratoire 21 est raccordée, en amont, fluidiquement à un port de sortie 51 du ventilateur médical 50, tel un connecteur, raccord ou analogue, de manière à récupérer et acheminer le gaz à base d'oxygène, typiquement de l'air ou mélange N₂/O₂ (contenant environ 21% d'O₂) délivré par le ventilateur médical 50, alors que la branche expiratoire 22 véhiculant les gaz expirés est raccordée fluidiquement à un port d'entrée 52 du ventilateur médical 50, tel un connecteur, raccord ou analogue, de manière à retourner au ventilateur médical 50 tout ou partie du débit des gaz expirés par le patient.

La branche expiratoire 22 des gaz expirés peut comprendre un ou plusieurs composants optionnels, comme par exemple un dispositif d'élimination du CO₂ 35, i.e. un piège à CO₂, tel un bac à chaud ou autre, permettant d'éliminer le CO₂ présent dans les gaz expirés par le patient ou un filtre ou autre.

Un capteur de débit 25, par exemple du type à fil chaud ou à différentiel de pression, est agencé sur la ligne d'alimentation en gaz ou branche inspiratoire 21, entre le ventilateur 50 et l'humidificateur 30, et est relié au dispositif de délivrance de NO 1, via une ligne de mesure de débit 26. Cet agencement sert à mesurer le débit de gaz délivré par le ventilateur 50, tel de l'air ou un mélange N₂/O₂, et circulant dans la branche inspiratoire 21, en amont du site de 24 raccordement du conduit ou ligne d'injection 23 où se fait le mélange gazeux NO/N₂/O₂. Ceci permet de réguler plus efficacement la délivrance du flux de NO (i.e. N₂/O₂) par le dispositif de délivrance de NO 1 puisque les mesures de débit opérées par le capteur de débit 25 sont retournées, via la ligne de mesure de débit 26, aux moyens de pilotage de l'appareil de délivrance de NO 1.

Comme schématisé en Fig. 2, l'appareil de fourniture de NO 1 comprend, de manière classique, un boitier rigide, par exemple en polymère, traversé par un circuit de gaz interne 6, tel un conduit de gaz ou analogue, pour acheminer le flux de NO/N₂ amené par la (ou les) ligne d'amenée de gaz 33 qui est alimentée par les bouteilles de mélange de NO/N₂ 31. Le circuit de gaz interne 6 relie fluidiquement l'entrée (ou les entrées) de gaz 21 de l'appareil de fourniture de NO 1 à la ligne d'injection 23, via une sortie de NO 5 de l'appareil 1, de manière à convoyer le flux gazeux à base de NO entre eux. Le circuit de gaz interne 6 est agencé dans le boitier.

Des moyens de contrôle de débit, typiquement des moyens à valve 7, i.e. un (ou des) dispositif à valve(s), par exemple une électrovanne ou une pluralité d'électrovannes agencées en parallèle, préférentiellement une ou des (électro)vannes proportionnelles, sont agencés sur le circuit de gaz interne 6, typiquement un passage de gaz interne, pour contrôler le (ou les) flux gazeux, i.e. le débit, qui y circule en direction de la ligne d'injection 24.

Pour ce faire, les moyens à valve 7 sont commandés par des moyens de pilotage 8, i.e. un (ou des) dispositif de pilotage ou contrôleur, aussi agencés dans le boitier de l'appareil de fourniture de NO 1, typiquement une carte électronique comprenant un (ou plusieurs) microprocesseur(s) 9, typiquement un (ou des) microcontrôleur, mettant en œuvre un ou des algorithmes. Ils peuvent comprendre d'autres éléments, tels des moyens de mémorisation (non montrés), comme un (ou des) mémoire informatique, telle une mémoire flash. Les moyens de pilotage 8 permettent notamment d'ajuster ou contrôler le débit de gaz en pilotant les moyens à valve, typiquement d'ouvrir ou fermer cette ou ces vannes, pour obtenir un débit de gaz déterminé et/ou calculé par les moyens de pilotage 8 à partir d'une valeur réglée/fixée par l'utilisateur, et en fonction du débit de gaz, i.e. air, délivré par le ventilateur 50 et mesuré par le capteur de débit 25 agencé sur la branche inspiratoire 21 et relié au dispositif de fourniture de NO 1, par la ligne de mesure de débit 26.

Le circuit de gaz interne 6, en particulier un passage de gaz interne, de l'appareil de fourniture de NO 1 peut aussi comprendre un (ou des) débitmètre (non montré) et/ou un régulateur de pression, tel un détendeur, (non montré) agencé en amont et/ou en aval des moyens à valve 7, pour déterminer le débit de gaz à base de NO circulant dans l'appareil de fourniture de NO 1. Le débitmètre peut être du type à différentiel de pression, à fil chaud ou autre. Il coopère avec les moyens de pilotage pour leur fournir, là encore, des mesures de débit du flux de NO/N₂, lesquelles mesures sont traitées par les moyens de pilotage 8 afin d'assurer une délivrance efficace de NO en fonction notamment du débit de gaz à base d'O₂ fourni par le ventilateur médical 50.

Habituellement, l'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections ou encore à entrer des informations, telles des valeurs désirées (e.g. débit, dosage de NO...), ou toute autre information ou donnée utile au personnel soignant. De préférence, l'affichage est en couleurs mais il peut se faire aussi en noir et blanc.

Les moyens de pilotage 8 de l'appareil de fourniture de NO 1 comprenant par exemple une carte de commande électronique et une unité de contrôle à microprocesseur 9, typiquement un microcontrôleur ou analogue. Les moyens de pilotage 8 permettent de contrôler ou commander tous les éléments électromécaniques de l'appareil 1. Plus précisément, la carte de commande intègre préférentiellement l'unité de contrôle et est configurée pour commander et par ailleurs analyser les signaux provenant des différents composants, tels que les capteurs...

L'alimentation électrique de l'appareil de fourniture de NO 1, en particulier des composants nécessitant du courant électrique pour fonctionner, tels les moyens de pilotage, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

Enfin l'installation 100 comprend aussi une ligne de prélèvement de gaz 60 qui relie fluidiquement la branche inspiratoire 21 à l'appareil de fourniture de NO 1. Elle vient se raccorder fluidiquement (en 61) à la ligne d'alimentation en gaz 21, entre l'humidificateur 30 et la pièce de jonction 25, i.e. pièce en Y, typiquement à proximité immédiate de la pièce de jonction 25, et par ailleurs à un port d'entrée 62 du dispositif de fourniture de NO 1, par exemple un port 62 porté par un connecteur, raccord ou analogue, permettant le raccordement de la ligne de prélèvement de gaz 60, tel un tuyau flexible ou analogue, à une ligne d'analyse de gaz 111 munie de capteurs 112 d'un analyseur de gaz interne 110, comme détaillé ci-après.

La ligne de prélèvement de gaz 60 permet de prélever dans la branche inspiratoire 21 du circuit patient 20 des échantillons de gaz et de les convoyer jusqu'au dispositif de fourniture de NO 1 où ils sont analysés dans un analyseur de gaz interne 110, c'est-à-dire au sein d'une ligne d'analyse de gaz 111 comprenant des moyens de mesure, tels un ou des capteurs 112, typiquement une ou des cellules électrochimiques par exemple, relié électriquement aux moyens de pilotage 8, afin de vérifier la conformité des échantillons gazeux analysés.

En particulier, il convient de vérifier que la composition du gaz final est conforme à celle du mélange gazeux NO/N₂/O₂ souhaité devant être administré au patient, notamment pour s'assurer qu'il ne contient pas de quantité excessive d'espèces NO₂ toxiques, que sa teneur en oxygène n'est pas hypoxique, qu'il ne contient pas une teneur en NO₂ trop élevée et que sa teneur en NO correspond à la posologie souhaitée, i.e. dose de NO à administrer par inhalation qui est habituellement choisie par le personnel soignant, i.e. médecin ou analogue.

Autrement dit, ligne de prélèvement de gaz 60 permet d'opérer un monitorage de la composition du mélange gazeux final, i.e. du mélange gazeux NO/N₂/O₂, afin de garantir que les teneurs en espèces NO et O₂ sont conformes à celles souhaitées et que celle en espèces NO₂ toxiques n'excède pas quelques ppmv.

Cette vérification de conformité se fait classiquement au moyen de moyens de mesure dédiés, typiquement des capteurs 112 de NO₂, de NO et d'O₂, par exemple des cellules électrochimiques ou analogues, qui doivent eux-mêmes être calibrés périodiquement, par exemple toutes les semaines. Cette calibration se fait en lançant une procédure de calibration prédéfinie et mémorisée dans les moyens de mémorisation de l'appareil 1, telle une mémoire flash ou analogue.

Les moyens de pilotage 8 de l'appareil 1 sont en outre configurés pour récupérer et traiter, i.e. analyser, les signaux provenant des différents capteurs 112 de l'analyseur de gaz 110, lequel est agencé dans l'appareil 1, et d'agir en réponse à ces signaux, notamment pour opérer la calibration des capteurs.

Selon l'invention, afin de minimiser le risque que les personnels de santé, c'est-à-dire la ou les équipes de soin, tels que médecin, infirmières ou autres, omettent de lancer tout ou partie des vérifications indispensables au bon fonctionnement de l'appareil de fourniture de NO 1, en particulière la calibration régulière des capteurs 112, c'est-à-dire des cellules de monitorage, typiquement des capteurs 112 de NO et NO₂, de la ligne d'analyse de gaz 111.

Pour ce faire, les moyens de pilotage 8, qui sont reliés électriquement au ou aux capteurs 112 de la ligne d'analyse de gaz 111, i.e. un ou des capteurs de NO et NO₂, sont configurés pour commander, à une fréquence temporelle donnée, par exemple une fois par jour, i.e. toutes les 24 heures, un affichage sur l'afficheur graphique 4, d'au moins une information relative à un ou plusieurs contrôles de sécurité à réaliser, i.e. à effectuer, typiquement un rappel d'une calibration à réaliser, et d'une première touche de sélection virtuelle 81, i.e. une première touche tactile, dont l'actionnement par l'utilisateur, c'est-à-dire le personnel soignant, engendre un lancement par les moyens de pilotage 8, d'une procédure de contrôle de sécurité, i.e. une procédure mémorisée, choisie parmi celle ou celles qui sont affichées par l'afficheur graphique 4, en particulier une procédure de calibration à effectuer préférentiellement quotidiennement, c'est-à-dire au moins 1 fois par jour.

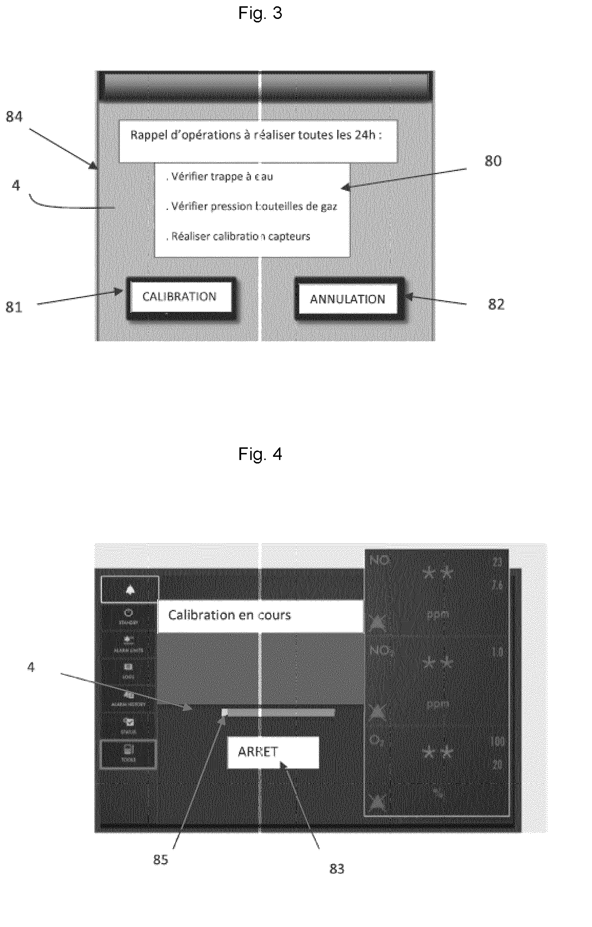

Fig. 3 représente un exemple d'affichage d'une information 80 relative des contrôles de sécurité à réaliser, à savoir ici vérifier la trappe à eau de l'appareil 1 et la pression du gaz dans les bouteilles, en particulier la pression du mélange gazeux NO/N₂, et opérer une calibration des capteurs de la ligne d'analyse de gaz, et d'une première touche de sélection virtuelle 81 (touche CALIBRATION), sur l'afficheur graphique 4 de l'appareil de fourniture de NO 1, servant à lancer une procédure de calibration, ainsi que préférentiellement une seconde touche de sélection virtuelle 82 (touche ANNULATION) servant ici à différer ou annuler le lancement, i.e. démarrage, de la procédure de calibration.

Préférentiellement, ces affichages se font au sein d'une fenêtre éphémère 84, aussi appelée 'pop-up' en anglais, apparaissant sur l'afficheur graphique 4.

L'affichage sur l'afficheur graphique 4 de cette ou ces informations 80 relatives à des contrôles de sécurité à réaliser, en particulier la calibration des capteurs, et des première et seconde touches de sélection virtuelle 81, 82 se fait automatiquement à une fréquence temporelle donnée, typiquement au moins 1 fois par jour, c'est-à-dire au moins toutes les 24h. Ceci est opéré par les moyens de pilotage 8, lesquels intègrent ou coopèrent avec un compteur temporel, par exemple intégré au microprocesseur 9.

Les informations 80 relatives à des contrôles de sécurité à réaliser sont préférentiellement mémorisées dans les moyens de mémorisation de l'appareil 1, telle une mémoire flash ou toute autre type de mémoire informatique.

Lorsque l'utilisateur voit apparaître, c'est-à-dire s'afficher, sur l'afficheur graphique 4, la ou les informations 80 relatives aux contrôles de sécurité à réaliser, en particulier la calibration des capteurs, et les première et éventuellement seconde touches de sélection virtuelle 81, 82, notamment au sein d'une fenêtre éphémère (i.e. « pop-up »), il peut décider :
- soit de différer ou empêcher/refuser le lancement de la procédure de calibration en appuyant sur la seconde touche de sélection virtuelle 82 qui est configurée pour empêcher, i.e. ne pas lancer la procédure de calibration,
- soit lancer la procédure de calibration en appuyant sur la première touche de sélection virtuelle 81.

De là, s'il appuie sur la première touche de sélection virtuelle 81, alors un premier signal (i.e. signal de lancement de calibration) est transmis aux moyens de pilotage 8 qui, en réponse à cet appui/sélection par l'utilisateur, commandent alors un arrêt de l'affichage de la ou les informations 80 relatives aux contrôles de sécurité à réaliser, e.g. la calibration des capteurs, et des première et seconde touches de sélection virtuelle 81, 82, et initient par ailleurs une procédure de calibration des capteurs de la ligne d'analyse de gaz de l'appareil 1.

Dans ce cas, la ou les informations 80 relatives aux contrôles de sécurité à réaliser, e.g. la calibration des capteurs, et les première et éventuellement seconde touches ne seront réaffichées qu'à l'issue de la fréquence temporelle donnée, par exemple 1 à 4 fois par jour, en particulier au sein d'une nouvelle fenêtre éphémère.

Toutefois, selon un mode de réalisation illustré en Fig. 4, une fois qu'une procédure de calibration des capteurs a été lancée, après appui sur la première touche de sélection virtuelle 81, l'appareil 1 peut être configuré pour afficher sur l'afficheur graphique 4, une troisième touche de sélection virtuelle 83 (touche ARRET), également actionnable par appui de l'utilisateur, dont l'actionnement (i.e. appui) engendre un signal d'annulation de procédure de calibration qui est transmis aux moyens de pilotage 8 qui, en réponse à ce signal d'annulation, commandent alors un arrêt de la procédure de calibration ayant débuté. Ceci permet donc à l'utilisateur d'arrêter une procédure de calibration par exemple s'il s'est trompé en lançant involontairement une telle procédure ou si, par ailleurs, la procédure doit être interrompue du fait d'un besoin urgent de l'appareil 1.

En d'autres termes, les moyens de pilotage peuvent en outre être configurés pour commander un affichage sur l'afficheur graphique 4 d'une troisième touche de sélection virtuelle 83 dont l'actionnement par l'utilisateur engendre un arrêt d'une procédure de calibration ayant débuté, c'est-à-dire ayant commencé après appui par l'utilisateur sur la première touche de sélection virtuelle.

Avantageusement, on affiche aussi un graphique temporel 85 de type barographe, sablier ou analogue, représentant le temps s'étant écoulé depuis le début de la procédure de calibration et/ou le temps restant avant terminaison, i.e. fin, de la procédure de calibration. Ceci permet d'aider l'utilisateur à déterminer s'il doit ou non interrompre la procédure de calibration en appuyant sur la troisième touche de sélection virtuelle 83.

A l'inverse, si l'utilisateur appuie sur la seconde touche de sélection virtuelle 82, alors un second signal (i.e. signal de non-lancement de calibration ou de calibration différée) est transmis aux moyens de pilotage 8 qui, en réponse à cet appui/sélection par l'utilisateur, commandent alors un arrêt de l'affichage de la ou les informations 80 relatives aux contrôles de sécurité à réaliser, e.g. la calibration des capteurs, et les première et seconde touches de sélection virtuelle 81, 82, mais sans lancer de calibration.

Dans ce cas, la ou les informations 80 relatives aux contrôles de sécurité à réaliser, e.g. la calibration des capteurs, et les première et préférentiellement seconde touches de sélection virtuelle 81, 82 disparaissent alors de l'écran d'affichage 4 et n'y seront réaffichées qu'après une durée donnée, par exemple après 1 ou 2 heures, ou une autre durée, ou alors uniquement à la fréquence temporelle donnée, c'est-à-dire 24h après par exemple.

L'affichage sur l'écran d'affichage 4 peut se faire en couleurs ou en noir et blanc, selon ce qui est sélectionné par l'utilisateur dans les menus de l'appareil 1.

L'appareil de fourniture de NO 1 de l'invention est utilisable dans une installation d'administration de gaz 100, comme par exemple celle de Fig. 1, pour administrer par inhalation, via un masque ou une sonde d'intubation trachéale, du monoxyde d'azote (NOi), i.e. le mélange final obtenu NO/O₂/N₂, à une ou des personnes, i.e. patients, en ayant besoin pour traiter leur pathologie pulmonaire, typiquement un ou des patients souffrant d'hypertension artérielle pulmonaire aiguë, notamment pour opérer une dilatation de leurs vaisseaux pulmonaires et/ou une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

## Revendications

1. Appareil de fourniture (1) d'un gaz contenant du NO comprenant :
- un circuit de gaz interne (6) pour acheminer un flux de gaz contenant du NO, comprenant des moyens à valve (7),
- des moyens de pilotage (8) à microprocesseur (9),
- un afficheur graphique (4) tactile commandé par les moyens de pilotage (8), et
- une ligne d'analyse de gaz (111) comprenant au moins un capteur (112) relié électriquement aux moyens de pilotage (8),
**caractérisé en ce que** les moyens de pilotage (8) sont configurés pour commander, à une fréquence temporelle donnée, un affichage temporaire sur l'afficheur graphique (4) d'au moins une première touche de sélection virtuelle (81) dont l'actionnement par un utilisateur engendre un lancement, par les moyens de pilotage (8), d'une procédure de calibration du ou des capteurs (112) de la ligne d'analyse de gaz (111).

2. Appareil selon la revendication 1, **caractérisé en ce que** la première touche de sélection virtuelle (81) coopère avec les moyens de pilotage (8), pour leur fournir au moins un signal de commande correspondant à un choix opéré par l'utilisateur correspondant au lancement de la procédure de calibration des capteurs.

3. Appareil selon la revendication 1, **caractérisé en ce que** les moyens de pilotage (8) sont en outre configurés pour commander un affichage sur l'afficheur graphique (4) d'au moins une information (80) relative à un ou plusieurs contrôles de sécurité à réaliser, notamment de la procédure de calibration des capteurs.

4. Appareil selon la revendication 3, **caractérisé en ce que** les moyens de pilotage (8) sont configurés pour commander l'affichage de ladite au moins une information (80) et/ou de la première touche de sélection virtuelle (81) à une fréquence temporelle d'au moins 1 fois par jour.

5. Appareil selon la revendication 1, **caractérisé en ce que** les moyens de pilotage (8) sont configurés pour commander, en outre, un affichage d'une seconde touche de sélection virtuelle (82) simultanément ou successivement à ladite première touche de sélection virtuelle (81), ladite seconde touche de sélection virtuelle (82) étant configurée pour annuler ou refuser ladite procédure de calibration des capteurs, en réponse à un actionnement par l'utilisateur de la seconde touche de sélection virtuelle (82).

6. Appareil selon les revendications 1, 3 et 5, **caractérisé en ce que** les moyens de pilotage (8) sont configurés pour commander un affichage simultané de ladite au moins une information (80) et des première et éventuellement seconde touches de sélection virtuelles (81, 82).

7. Appareil selon la revendication 1, **caractérisé en ce que** les moyens de pilotage (8) commandent les moyens à valve (7) pour contrôler le flux de gaz dans le circuit de gaz interne (6), de manière à autoriser ou stopper toute circulation de gaz dans ledit circuit de gaz interne (6).

8. Appareil selon la revendication 1, **caractérisé en ce que** ledit au moins un capteur (112) comprend une ou des cellules électrochimiques.

9. Appareil selon la revendication 6, **caractérisé en ce que** les moyens de pilotage (8) sont configurés pour commander l'affichage simultané de ladite au moins une information (80) et des première et éventuellement seconde touches de sélection virtuelles (81, 82), au sein d'une fenêtre d'affichage éphémère (84).

10. Appareil selon la revendication 4, **caractérisé en ce que** les moyens de pilotage (8) sont configurés pour commander l'affichage de ladite au moins une information (80) et/ou de la première touche de sélection virtuelle (81) à une fréquence temporelle de 1 à 4 fois par jour, de préférence 1 ou 2 fois par jour.

11. Appareil selon la revendication 3, **caractérisé en ce qu'**il comprend des moyens de mémorisation () configurés pour mémoriser la ou les procédures de contrôle de sécurité, en particulier la procédure de calibration des capteurs.

12. Appareil selon la revendication 5, **caractérisé en ce que** les moyens de pilotage (8) sont en outre configurés pour commander un affichage sur l'afficheur graphique (4) d'une troisième touche de sélection virtuelle (83) dont l'actionnement par l'utilisateur engendre un arrêt d'une procédure de calibration ayant débuté.

13. Appareil selon la revendication 1, **caractérisé en ce que** les moyens de pilotage (8) sont en outre configurés pour commander un affichage sur l'afficheur graphique (4) d'un graphique temporel (85) représentant le temps s'étant écoulé depuis le début de la procédure de calibration et/ou le temps restant avant terminaison de la procédure de calibration, en particulier un graphique temporel représentant un barographe ou un sablier.

14. Appareil selon les revendications 12 et 13, **caractérisé en ce que** les moyens de pilotage (8) sont en outre configurés pour opérer un affichage simultané sur l'afficheur graphique (4), de la troisième touche de sélection virtuelle (83) et du graphique temporel (85).

15. Installation d'administration de gaz (100) gaz à un patient comprenant :
- au moins une source de gaz (10) contenant du NO gazeux, en particulier un mélange gazeux NO/N₂,
- un appareil de fourniture de gaz (1) selon l'une des revendications précédentes, alimenté en gaz contenant du NO par ladite au moins une source de gaz (10),
- un ventilateur médical (50) pour fournir un gaz contenant de l'oxygène et
- une ligne d'alimentation (21) alimentée en gaz par l'appareil de fourniture de gaz (1) et par le ventilateur médical (50).

## Patentansprüche

1. Vorrichtung (1) zur Zufuhr eines NO-haltigen Gases, umfassend:
- einen internen Gaskreislauf (6) zum Leiten eines NO-haltigen Gasstroms, der Ventilmittel (7) umfasst,
- Steuermittel (8) mit Mikroprozessor (9),
- eine von der Steuereinrichtung (8) gesteuerte grafische Touchscreen-Anzeige (4) und
- eine Gasanalyseleitung (111) mit mindestens einem Sensor (112), der elektrisch mit den Steuermitteln (8) verbunden ist,
**dadurch gekennzeichnet, dass** die Steuermittel (8) so konfiguriert sind, dass sie mit einer bestimmten Zeitfrequenz eine temporäre Anzeige auf dem Grafikdisplay (4) mindestens einer ersten virtuellen Auswahltaste (81) steuern, deren Betätigung durch einen Benutzer durch die Steuermittel (8) einen Start eines Kalibrierungsvorgangs des oder der Sensoren (112) der Gasanalyseleitung (111) bewirkt.

2. Vorrichtung nach Anspruch 1, **dadurch gekennzeichnet, dass** die erste virtuelle Auswahltaste (81) mit den Steuermitteln (8) zusammenwirkt, um ihnen mindestens ein Steuersignal zu liefern, das einer vom Benutzer getroffenen Auswahl entspricht, die dem Start des Kalibrierungsvorgangs der Sensoren entspricht.

3. Vorrichtung nach Anspruch 1, **dadurch gekennzeichnet, dass** die Steuermittel (8) außerdem so konfiguriert sind, dass sie auf dem Grafikdisplay (4) mindestens eine Information (80) über eine oder mehrere durchzuführende Sicherheitskontrollen, insbesondere des Kalibrierungsverfahrens der Sensoren, anzeigen.

4. Vorrichtung nach Anspruch 3, **dadurch gekennzeichnet, dass** die Steuermittel (8) so konfiguriert sind, dass sie die Anzeige der mindestens einen Information (80) und/oder der ersten virtuellen Auswahltaste (81) mit einer zeitlichen Frequenz von mindestens einmal pro Tag steuern.

5. Vorrichtung nach Anspruch 1, **dadurch gekennzeichnet, dass** die Steuermittel (8) so konfiguriert sind, dass sie zusätzlich eine Anzeige einer zweiten virtuellen Auswahltaste (82) gleichzeitig oder nacheinander mit der ersten virtuellen Auswahltaste (81) steuern, wobei die zweite virtuelle Auswahltaste (82) so konfiguriert ist, dass sie den Kalibrierungsvorgang der Sensoren abbricht oder ablehnt, wenn der Benutzer die zweite virtuelle Auswahltaste (82) betätigt.

6. Vorrichtung nach den Ansprüchen 1, 3 und 5, **dadurch gekennzeichnet, dass** die Steuermittel (8) so konfiguriert sind, dass sie eine gleichzeitige Anzeige der mindestens einen Information (80) und der ersten und gegebenenfalls zweiten virtuellen Auswahltasten (81, 82) steuern.

7. Vorrichtung nach Anspruch 1, **dadurch gekennzeichnet, dass** die Steuermittel (8) die Ventilmittel (7) so steuern, dass sie den Gasfluss im internen Gaskreislauf (6) so regeln, dass jeglicher Gasfluss im internen Gaskreislauf (6) freigegeben oder gestoppt wird.

8. Vorrichtung nach Anspruch 1, **dadurch gekennzeichnet, dass** der mindestens eine Sensor (112) eine oder mehrere elektrochemische Zellen umfasst.

9. Vorrichtung nach Anspruch 6, **dadurch gekennzeichnet, dass** die Steuermittel (8) so konfiguriert sind, dass sie die gleichzeitige Anzeige der mindestens einen Information (80) und der ersten und gegebenenfalls zweiten virtuellen Auswahltasten (81, 82) in einem kurzzeitigen Anzeigefenster (84) steuern.

10. Vorrichtung nach Anspruch 4, **dadurch gekennzeichnet, dass** die Steuermittel (8) so konfiguriert sind, dass sie die Anzeige der mindestens einen Information (80) und/oder der ersten virtuellen Auswahltaste (81) mit einer Zeitfrequenz von 1 bis 4 Mal pro Tag, vorzugsweise 1 oder 2 Mal pro Tag, steuern.

11. Vorrichtung nach Anspruch 3, **dadurch gekennzeichnet, dass** sie Speichermittel () umfasst, die so konfiguriert sind, dass sie das oder die Sicherheitsprüfverfahren, insbesondere das Kalibrierungsverfahren der Sensoren, speichern.

12. Vorrichtung nach Anspruch 5, **dadurch gekennzeichnet, dass** die Steuermittel (8) außerdem so konfiguriert sind, dass sie auf dem Grafikdisplay (4) eine dritte virtuelle Auswahltaste (83) anzeigen, deren Betätigung durch den Benutzer einen bereits begonnenen Kalibrierungsvorgang stoppt.

13. Vorrichtung nach n Anspruch 1, **dadurch gekennzeichnet, dass** die
Steuermittel (8) außerdem so konfiguriert sind, dass sie auf dem Grafikdisplay (4) eine Zeitgrafik (85) anzeigen, die die seit Beginn des Kalibrierungsverfahrens verstrichene Zeit und/oder die bis zum Ende des Kalibrierungsverfahrens verbleibende Zeit darstellt, insbesondere eines Zeitdiagramms, das einen Barographen oder eine Sanduhr darstellt.

14. Vorrichtung nach den Ansprüchen 12 und 13, **dadurch gekennzeichnet, dass** die Steuermittel (8) außerdem so konfiguriert sind, dass sie gleichzeitig auf der grafischen Anzeige (4) die dritte virtuelle Auswahltaste (83) und die Zeitgrafik (85) anzeigen.

15. Gasversorgungsanlage (100) zur Versorgung eines Patienten mit Gas, umfassend:
- mindestens eine Gasquelle (10), die gasförmiges NO enthält, insbesondere ein NO/N₂-Gasgemisch ,
- eine Gasversorgungsvorrichtung (1) gemäß einem der vorstehenden Ansprüche, die von der mindestens einen Gasquelle (10) mit NO-haltigem Gas versorgt wird,
- einem medizinischen Ventilator (50) zur Zufuhr von sauerstoffhaltigem Gas und
- eine Versorgungsleitung (21), die von der Gasversorgungsvorrichtung (1) und dem medizinischen Ventilator (50) mit Gas versorgt wird.

## Claims

1. Apparatus (1) for supplying a NO-containing gas, comprising:
- an internal gas circuit (6) for conveying a flow of NO-containing gas, comprising valve means (7),
- microprocessor-based control means (8),
- a touchscreen graphic display (4) controlled by the control means (8), and
- a gas analysis line (111) comprising at least one sensor (112) electrically connected to the control means (8),
**characterized in that** the control means (8) are configured to control, at a given time frequency, a temporary display on the graphic display (4) of at least one first virtual selection key (81) whose actuation by a user causes the control means (8) to launch of a calibration procedure for the sensor or sensors (112) of the gas analysis line (111).

2. Apparatus according to claim 1, **characterized in that** the first virtual selection key (81) cooperates with the control means (8) to provide them with at least one control signal corresponding to a choice made by the user corresponding to the launch of the sensor calibration procedure.

3. Apparatus according to claim 1, **characterized in that** the control means (8) are further configured to control the display on the graphic display (4) of at least one piece of information (80) relating to one or more safety checks to be carried out, in particular of the sensor calibration procedure.

4. Apparatus according to claim 3, **characterized in that** the control means (8) are configured to control the display of said at least one piece of information (80) and/or the first virtual selection key (81) at a frequency of at least once per day.

5. Apparatus according to claim 1, **characterized in that** the control means (8) are configured to control, in addition, the display of a second virtual selection key (82) simultaneously or successively with said first virtual selection key (81), said second virtual selection key (82) being configured to cancel or refuse said sensor calibration procedure in response to the user activating the second virtual selection key (82).

6. Apparatus according to claims 1, 3, and 5, **characterized in that** the control means (8) are configured to control simultaneous display of said at least one piece of information (80) and the first and possibly second virtual selection keys (81, 82).

7. Apparatus according to claim 1, **characterized in that** the control means (8) control the valve means (7) to control the flow of gas in the internal gas circuit (6) so as to allow or stop any circulation of gas in said internal gas circuit (6).

8. Apparatus according to claim 1, **characterized in that** said at least one sensor (112) comprises one or more electrochemical cells.

9. Apparatus according to claim 6, **characterized in that** the control means (8) are configured to control the simultaneous display of said at least one piece of information (80) and the first and possibly second virtual selection keys (81, 82) within a temporary display window (84).

10. Apparatus according to claim 4, **characterized in that** the control means (8) are configured to control the display of said at least one piece of information (80) and/or the first virtual selection key (81) at a frequency of 1 to 4 times per day, preferably 1 or 2 times per day.

11. Apparatus according to claim 3, **characterized in that** it comprises storage means () configured to store the safety check procedure or procedures, in particular the sensor calibration procedure.

12. Apparatus according to claim 5, **characterized in that** the control means (8) are further configured to control the display on the graphic display (4) of a third virtual selection key (83) whose actuation by the user causes a calibration procedure that has started to be stopped.

13. Apparatus according to claim 1, **characterized in that** the control means (8) are further configured to control the display on the graphic display (4) of a time graph (85) representing the time elapsed since the start of the calibration procedure and/or the time remaining before the end of the calibration procedure, in particular a time graph representing a barograph or an hourglass.

14. Apparatus according to claims 12 and 13, **characterized in that** the control means (8) are further configured to simultaneously display the third virtual selection key (83) and the time graph (85) on the graphic display (4).

15. Gas administration device (100) for administering gas to a patient, comprising:
- at least one gas source (10) containing gaseous NO, in particular a NO/N₂gas mixture,
- a gas supply apparatus (1) according to one of the preceding claims, supplied with NO-containing gas by said at least one gas source (10),
- a medical ventilator (50) for supplying oxygen-containing gas, and
- a supply line (21) supplied with gas by the gas supply device (1) and by the medical ventilator (50).
